# EUROPEAN PATENT APPLICATION

(11) **EP 1 894 568 A1**
(43) Date of publication of application: **05.03.2008**
(21) Application number: 06119917.0
(22) Date of filing: 31.08.2006
(51) Int. Cl.: A61K 31/58, A61K 31/40, A61K 31/167, A61K 31/137, A61P 11/00, A61P 11/06, A61P 43/00

(54) **Pharmaceutical compositions for the treatment of inflammatory or obstructive airway diseases**

(71) Applicant: Novartis AG, 4056 Basel (CH)
(72) Inventor: The designation of the inventor has not yet been filed
(74) Representative: McLean, Craig Sutherland

(57) **Abstract**

Medicaments comprising (A) an antimuscarinic agent, (B) a beta-2 adrenoreceptor agonist and (C) a corticosteroid for the treatment of inflammatory or obstructive airways diseases.

## Description

This invention relates to organic compounds and their use as pharmaceuticals, in particular for the treatment of inflammatory or obstructive airways diseases.

In one aspect, the present invention provides a medicament, (Group I), comprising, separately or together
(A) a glycopyrronium salt;
(B) a beta-2 adrenoceptor agonist selected from salmeterol and formoterol, or pharmaceutically acceptable salts thereof and
(C) a corticosteroid selected from fluticasone propionate, 4-Methyl-thiazole-5-carboxylic acid (6S,9R,10S,11S,13S,16R,17R)-6,9-difluoro-17 fluoromethylsulfanylcarbonyl-11-hydroxy-10,13,16-trimethyl-3-oxo-6,7,8,9,10,11,12,13,14,15,16,17-dodecahydro-3H-cyclopenta[a]phenanthren-17-yl ester, Furan-2-carboxylic acid (6S,9R,10S,11S,13S,16R,17R)-6,9-difluoro-17-fluoromethylsulfanylcarbonyl-11-hydroxy-10,13,16-trimethyl-3-oxo-6,7,8,9,10,11,12,13,14,15,16,17-dodecahydro-3H-cyclopenta[a]phenanthren-17-yl ester, and budesonide;
for simultaneous, sequential or separate administration in the treatment of an inflammatory or obstructive airways disease.

A glycopyrronium salt includes glycopyrronium bromide, or glycopyrrolate, and is an antimuscarinic agent that is currently administered by injection to reduce secretions during anaesthesia and or taken orally to treat gastric ulcers. Schroeckenstein et al J. Allergy Clin. Immunol. 1998; 82(1): 115-119 discloses the use of glycopyrrolate in an aerosol formulation for treating asthma where a single administration of a metered dose achieved bronchodilation for up to 12 hours. More recently international patent application WO 2001/76575 discloses glycopyrrolate can be formulated for pulmonary delivery in controlled release formulation that permits the antimuscarinic agent to exert its pharmacological effect over a period greater than 12 hours.

Salmeterol or a pharmaceutically acceptable salt form thereof possess beta-2 adrenoceptor agonist activity as described in as described in U.S. Pat. Nos. 4,992,474, 5,126,375, and 5,225,445. They commonly have a rapid onset of action and have a prolonged stimulating action on the β₂-adrenoceptor, for example up 24 hours or longer. They may be prepared by using the procedures described in U.S. Pat. Nos. 4,992,474, 5,126,375, and 5,225,445.

Formoterol or a pharmaceutically acceptable salt thereof, possess beta-2 adrenoceptor agonist activity as described in as described in U.S. Pat. No. 3,994,974 or U.S. Pat. No. 5,684,199.

Fluticasone propionate is an anti-inflammatory corticosteroid and is described in U.S. Pat. No. 4,335,121.

Budesonide is an anti-inflammatory corticosteroid and is described in U.S. Pat. No. 3,929,768.

In another aspect of the invention provides a medicament, (Group II), comprising, separately or together
(A) compounds of form ula I 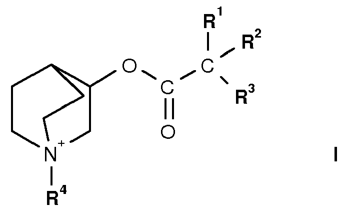 in salt or zwitterionic form wherein
   R¹ and R³ are each independently a C₃-C₁₅-carbocyclic group, C₆-C₁₅-aromatic carbocyclic group, or a 4- to 12 -membered heterocyclic group having at least one ring heteroatom selected from nitrogen, oxygen and sulphur,
   or -CR¹R²R³ together form a group of formula 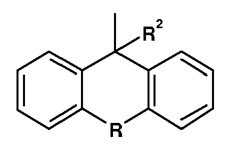 where R is a bond, -O-, -S- , -CH2-, -CH=CH-, -CH₂-CH₂-, amino or -N(CH₃)-;
   R² is hydrogen, halo, hydroxy, C₁-C₈-alkoxy or C₁-C₈-alkyl optionally substituted by hydroxy; R⁴ is C₁C₈-alkyl substituted by NHR⁵, -NR⁵-CO-R⁶, -NR⁵-CO-NH-R⁷, -NR⁵-SO₂-R⁸ -CONR⁹R¹⁰, -OR¹¹, -O-CO-NHR¹², -O-CO-R¹³ or -CO-O-R¹⁴, or R⁴ is C₃-C₁₀-alkynyl optionally substituted by a C₃-C₁₅-carbocyclic group, C₆-C₁₅-aromatic carbocyclic group, or a 4- to 12-membered heterocyclic group having at least one ring heteroatom selected from nitrogen, oxygen and sulphur;
   R⁵ is hydrogen or C₁-C₈-alkyl;
   R⁶ is C₁-C₈-alkyl, C₂-C₈-alkenyl, C₂-C₁₀-alkynyl or C₁-C₈-alkoxy in each case optionally substituted by a C₃-C₁₅-carbocyclic group, C₆-C₁₅-aromatic carbocyclic group, or a 4- to 12-membered heterocyclic group having at least one ring heteroatom selected from nitrogen, oxygen and sulphur, or R⁶ is a C₃-C₁₅-carbocyclic group, C₆-C₁₅-aromatic carbocyclic group, or a 4- to 12-membered heterocyclic group having at least one ring heteroatom selected from nitrogen, oxygen and sulphur;
   R⁷ is a C₃-C₁₅-carbocyclic group or C₆-C₁₅-aromatic carbocyclic group;
   R⁸ is a C₃-C₁₅-carbocylic group or C₆-C₁₅-aromatic carbocyclic group;
   R⁹ is hydrogen or C₁C₈-alkyl;
   R¹⁰ is hydrogen, C₁-C₈-alkyl optionally substituted by cyano, amino, nitro, carboxy, C₁-C₈-alkoxy, a C₃-C₁₅-carbocyclic group, C₆-C₁₅-aromatic carbocyclic group, or by a 4- to 12-membered heterocyclic group having at least one ring heteroatom selected from nitrogen, oxygen and sulphur, or R¹⁰ is a C₃-C₁₅-carbocyclic group, C₆-C₁₅-aromatic carbocyclic group, or a 4- to 12-membered heterocyclic group having at least one ring heteroatom selected from nitrogen, oxygen and sulphur;
   R¹¹ is hydrogen, C₁-C₈-alkyl, C₁-C₈-alkyl-C₁-C₈-alkoxy or C₁-C₈-alkyl-O-R¹⁵;
   R¹² is a C₃-C₁₅-carbocyclic group or C₆-C₁₅-aromatic carbocyclic group;
   R¹³ is C₁-C₈-alkyl or a C₃-C₁₅-carbocyclic group or C₆-C₁₅-aromatic carbocyclic group;
   R¹⁴ is hydrogen, a C₃-C₁₅-carbocylic group, C₆-C₁₅-aromatic carbocyclic group, C₁-C₈-alkenyl, or C₁-C₈-alkyl optionally substituted by a C₃-C₁₅-carbocylic group or C₆-C₁₅-aromatic carbocyclic group;
   R¹⁵ is a C₃-C₁₅-carbocyclic group or C₆-C₁₅-aromatic carbocyclic group; and
   where each C₃-C₁₅-carbocyclic group is optionally substituted by halo (e.g. fluoro, chloro or bromo), cyano, hydroxy, amino, nitro, carboxy, C₁-C₈-alkyl (e.g. methyl or ethyl), halo -C₁-C₈-alkyl, C₁-C₈-alkoxy, C₁-C₈-alkylcarbonyl, C₁-C₈-alkylsulfonyl, -SO₂NH₂, a C₃-C₁₅-carbocyclic group and a 4 - to 12-membered heterocyclic group having at least one ring heteroatom selected from nitrogen, oxygen and sulphur and each C₆-C₁₅-aromatic carbocyclic group is optionally substituted by halo (e.g. fluoro, chloro or bromo), cyano, hydroxy, amino, nitro, carboxy, C₁-C₈-alkyl (e.g. methyl or ethyl), halo-C₁-C₈-alkyl, C₁-C₈-alkoxy, C₁-C₈-alkylcarbonyl, C₁-C₈-alkylsulfonyl, -SO₂NH₂,a C₃-C₁₅-carbocyclic group and a 4-to 12-membered heterocyclic group having at least one ring heteroatom selected from nitrogen, oxygen and sulphur,
(B) a beta-2 adrenoceptor agonist selected from salmeterol and formoterol, or pharmaceutically acceptable salts thereof; and
(C) a corticosteroid selected from fluticasone propionate, 4-Methyl-thiazole-5-carboxylic acid (6S,9R,10S,11S,13S,16R,17R)-6,9-difluoro-17 fluoromethylsulfanylcarbonyl-11-hydroxy-10,13,16-trimethy]-3-oxo-6,7,8,9,10,11,12,13,14,15,16,17-dodecahydro-3H-cyclopenta[a]phenanthren-17-yl ester, Furan-2-carboxylic acid (6S,9R,10S,11S,13S,16R,17R)-6,9-difluoro-17-fluoromethylsulfanylcarbonyl-11-hydroxy-10,13,16-trimethyl-3-oxo-6,7,8,9,10,11,12,13,14,15,16,17-dodecahydro-3H-cyclopenta[a]phenanthren-17-yl ester, and budesonide; for simultaneous, sequential or separate administration in the treatment of an inflammatory or obstructive airways disease.

In another aspect, the present invention provides a medicament, (Group III), comprising, separately or together
(A) a glycopyrronium salt;
(B) a beta-2 adrenoceptor agonist selected from salmeterol and formoterol, or pharmaceutically acceptable salts thereof; and
(C) a corticosteroid selected from mometasone furoate and a compound of formula II 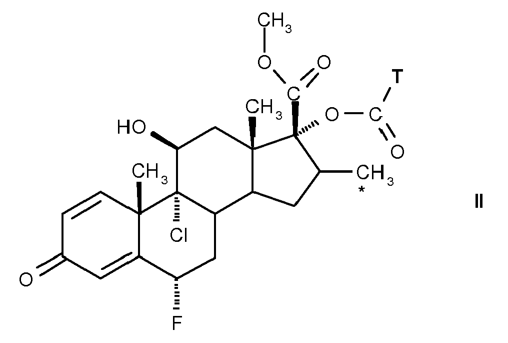 where T is a monovalent cyclic organic group having from 3 to 15 atoms in the ring system;
for simultaneous, sequential or separate administration in the treatment of an inflammatory or obstructive airways disease.

A glycopyrronium salt is as defined in Group I.

Mometasone furoate, (11β, 16a)-9,21-dichloro -17-[(2-furanylcarbonyl)oxy]-11-hydroxy-16-methylpregna-1, 4-diene-3,20-dione, alternatively designated 9α,21-dichloro-16α-methyl-1,4-pregnadiene-11β,17α-diol-3,20-dione 17-(2'-furoate), is an anti-inflammatory corticosteroid that is described in United States patent specification U.S. Pat. No. 4,472,393.

Compounds of formula II are disclosed, together with procedures for their preparation in international patent application WO 02/00679, the contents of which is incorporated herein by reference.

In another aspect, the present invention provides a medicament, (Group IV), comprising, separately or together
(A) compounds of formula I, as defined in Group II,
(B) a beta-2 adrenoceptor agonist selected from salmeterol and formoterol, or pharmaceutically acceptable salts thereof; and
(C) a corticosteroid selected from mometasone furoate and a compound of formula II 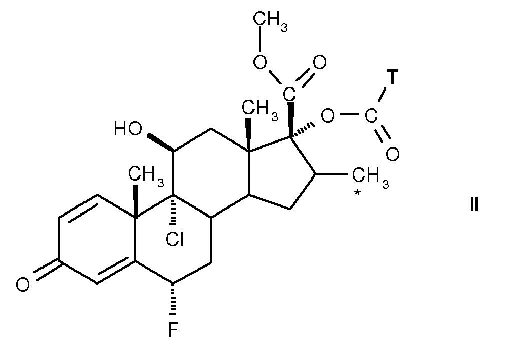 where T is a monovalent cyclic organic group having from 3 to 15 atoms in the ring system;

Compounds of formula I are as defined in (Group II).

Formoterol or a pharmaceutically acceptable salt thereof, as described in (Group III).

Mometasone furoate is as defined in (Group III).

Compounds of formula II are as defined in (Group III).

Terms used in the specification have the following meanings:
"Optionally substituted" means the group referred to can be substituted at one or more positions, e.g. 1, 2 or 3 positions, by any one or any combination of the radicals described.
"C₁-C₈-alkyl" as used herein denotes straight chain or branched alkyl having 1 to 8 carbon atoms. Preferably, C₁-C₈-alkyl is C₁-C₄-alkyl.
"C₁-C₈-alkylene" as used herein denotes straight chain or branched alkylene that contains 1 to 8 carbon atoms. Preferably, C₁-C₈-alkylene is C₁-C₄-alkylene.
"C₂-C₈-alkenyl" as used herein denotes straight chain or branched hydrocarbon chains that contain two to eight carbon atoms and one or more carbon-carbon double bonds. Preferably "C₂-C₈-alkenyl" is "C₂-C₄-alkenyl".
" C₂-C₁₀-alkynyl" as used herein denotes straight chain or branched hydrocarbon chains that contain two to ten carbon atoms and one or more carbon -carbon triple bonds. Preferably "C₂-Cio-alkynyl" is "C₃-C₈-alkynyl".
"C₃-C₁₅-Carbocyclic group", as used herein, denotes a carbocyclic group having 3- to 15-ring carbon atoms that is saturated or partially saturated, such as a C₃-C₈-cycloalkyl. Examples of C₃-C₁₅-Carbocyclic groups include but are not limited to cyclopropyl, cyclobutyl, cyclopentyl, cyclohexyl, cycloheptyl or cyclooctyl or a bicyclic group, such as bicyclooctyl, bicyclononyl including indanyl and indenyl, and bicyclodecyl.
"C₆-C₁₅-aromatic carbocyclic group", as used herein, denotes an aromatic group having 6- to 15-ring carbon atoms. Examples of C₆-C₁₅-aromatic carbocyclic groups include but are not limited to phenyl, phenylene, benzenetriyl, naphthyl, naphthylene, naphthalenetriyl or anthrylene.
" C₃-C₈-cycloalkyl" as used herein denotes cycloalkyl having 3 to 8 carbon atoms.
   Preferably "C₃-C₈-cycloalkyl" is "C₃-C₆-cycloalkyl".
" C₁-C₈-haloalkyl" as used herein denotes C₁-C₈-alkyl as hereinbefore defined substituted by one or more halogen atoms, preferably one, two or three halogen atoms. Preferably "C₁-C₈-haloalkyl" is "C₁-C₄-haloalkyl".
" C₁-C₈-alkylcarbonyl" as used herein denotes C₁-C₈-alkyl as hereinbefore defined linked to a carbonyl group. Preferably "C₁-C₈-alkylcarbonyl" is "C₁-C₄-alkylcarbonyl".
"C₁-C₈-alkylthio" as used herein denotes C₁-C₈-alkyl as hereinbefore defined linked to -S-. Preferably "C₁-C₈-alkylthio" is "C₁-C₄-alkylthio".
" C₁-C₈-alkylsulfonyl" as used herein denotes C₁-C₈-alkyl as hereinbefore defined linked to-SO₂-. Preferably " C₁-C₈-alkylsulfonyl" is "C₁-C₄-alkylsulfonyl".
"C₁-C₈-alkoxy" as used herein denotes straight chain or branched alkoxy having 1 to 8 carbon atoms. Preferably, C₁-C₈-alkoxy is C₁-C₄-alkoxy.
"C₁-C₈-haloalkoxy" as used herein denotes C₁-C₈-alkoxy as hereinbefore defined substituted by one or more halogen atoms, preferably one, two or three halogen atoms. Preferably "C₁-C₈-haloalkoxy" is "C₁-C₄-haloalkoxy".
   "di(C₁-C₈-alkyl)sulfamoyl" as used herein denotes -SO₂-NH₂ where the nitrogen atom is substituted at two positions by C₁-C₈-alkyl as hereinbefore defined, which may be the same or different. Preferably di(C₁-C₈-alkyl)sulfamoyl is -SO₂-N(CH₃)₂
"Halo" or "halogen" as used herein denotes a element belonging to group 17 (formerly group VII) of the Periodic Table of Elements, which may be, for example, fluorine, chlorine, bromine or iodine. Preferably halo or halogen is fluorine, chlorine or bromine.
"Aminocarbonyl" as used herein denotes amino attached through the nitrogen atom to a carbonyl group.
" 4- to 12-membered heterocyclic group containing at least one ring heteroatom selected from nitrogen, oxygen and sulphur" as used herein denotes a monoheterocyclic, biheterocyclic or triheterocyclic group, which may be saturated or unsaturated, that has 4 to 12 ring atoms. Monoheterocyclic groups include azetidinyl, tetrahydrofuranyl, furyl, pyrrolyl, pyrrolidinyl, pyrazolyl, imidazolyl, triazolyl, tetrazolyl, thienyl, thiazolyl, thiadiazolyl, isothiazolyl, oxadiazolyl, pyridinyl, oxazolyl, isoxazolyl, piperidinyl, pyridinyl, pyrazinyl, pyridazinyl, pyrimidinyl, piperazinyl, morpholinyl, triazinyl, oxazinyl, thiazolyl or tetrahydropyranyl. Biheterocyclic groups include thienothienyl, benzazolyl, benzothienyl, benzimidazolyl, benzodioxinyl, indazolyl, benzothiazolyl, imidazopyridinyl and naphthyridinyl. Preferred 4-to 12-membered heterocyclic groups in clude azetidinyl, tetrahydrofuranyl, furyl, pyrrolyl, pyrazolyl, triazolyl, thienyl, thiazolyl, thiadiazolyl, oxazolyl, isoxazolyl, tetrahydropyranyl, piperidinyl, pyridinyl, pyrazinyl, pyrimidinyl, thienothienyl, benzazolyl, benzothienyl, benzimidazolyl, benzodioxinyl, indazolyl and benzothiazolyl, imidazopyridinyl, naphthyridinyl. The 4- to 12-membered heterocyclic group can be unsubstituted or substituted at one or more positions, e.g. 1, 2 or 3 positions, by any one or any combination of substituents. Preferred substituents include halo (e.g. fluoro, chloro or bromo), cyano, oxo, hydroxy, carboxy, nitro, C₁-C₈-alkyl (e.g. methyl or ethyl), halo-C₁-C₈-alkyl (e.g. trifluoromethyl), C₁-C₈-alkylcarbonyl, di(C₁C₈-alkyl)sulfamoyl and C₁-C₈-alkoxy optionally substituted by aminocarbonyl.
   Especially preferred substituents include halo, oxo, C₁-C₄-alkyl and C₁-C₄-alkylcarbonyl.

Throughout this specification and in the claims that follow, unless the context requires otherwise, the word "comprise", or variations such as "comprises" or "comprising", will be understood to imply the inclusion of a stated integer or step or group of integers or steps but not the exclusion of any other integer or step or group of integers or steps.

Triple combinations comprising novel beta-2 adrenoceptor agonist s are described in patent applications GB 0523655.9 and GB 0523656.7.

It has now surprisingly been found that a significant unexpected therapeutic benefit, particularly a synergistic therapeutic benefit, in the treatment of inflammatory or obstructive airways diseases can be obtained by combination therapy using the compounds of (A), (B), and (C) of either (Group I), (Group II), (Group III) and (Group IV) respectively. For instance, it is possible using th ese combination therapies to reduce the dosages of one or more of the three active ingredients required for a given therapeutic effect considerably compared with those required using treatment with the active ingredients alone, thereby minimising possibly undesirable side effects. In particular, the amount of fluticasone propionate needed for a given anti-inflammatory effect may be significantly reduced when used in admixture with glycopyrronium bromide and salmeterol or a pharmaceutically acceptable salt form, or a compound of formula I and salmeterol or a pharmaceutically acceptable salt form, thereby reducing the risk of undesirable side effects from the repeated exposure to the steroid involved in the treatment of inflammatory or obstructive airways diseases.

Similarly, the amount of mometasone furoate needed for a given-inflammatory effect may be significantly reduced when used in admixture with glycopyrronium bromide and salmeterol or a pharmaceutically acceptable salt form, or a compound of formula I and salmeterol or a pharmaceutically acceptable salt form, thereby reducing the risk of undesirable side effects from the repeated exposure to the steroid involved in the treatment of inflammatory or obstructive airways diseases.

Furthermore, using the combination therapy of the invention, particularly using compositions containing glycopyrronium bromide, salmeterol or formoterol and fluticasone propionate, glycopyrronium bromide, salmeterol and budesonide, (R)-3-(2-Hydroxy-2,2-diphenyl-acetoxy)-1-(isoxazol-3-ylcarbamoyl-methyl)-1-azonia-bicyclo-[2.2.2]octanebromide (aquinuclidine), ((R)-3-((R)-2-cyclohexyl-2-hydroxy-2-phenylacetoxy)-1-(isoxazol-3-ylcarbamoylmethyl)-1-azoniabicyclo[2.2.2]octane or any of the examples in WO 2004/096800 and WO 2006/048225, salmeterolor formoterol and fluticasone propionate, or (R)-3-(2-Hydroxy-2,2-diphenyl-acetoxy)-1-(isoxazol-3-ylcarbamoyl-methyl)-1-azonia-bicyclo-[2.2.2]octane bromide (a quinuclidine), ((R)-3-((R)-2-cyclohexyl-2-hydroxy-2-phenylacetoxy)-1-(isoxazol-3-ylcarbamoylmethyl)-1-azoniabicyclo[2.2.2]octane or any of the examples in WO 2004/096800 and WO 2006/048225, salmeterol and budesonide, or glycopyrronium bromide, sameterol or formoterol fumarate, and mometasone furoate, glycopyrronium bromide, salmeterol or formoterol fumarate, and 3 Methyl-thiophene-2-carboxylic acid (6S,9R,10S,11S,13S,16R, 17R)-9-chloro-6-fluoro-11-hydroxy-17-methoxycarbonyl-10,13,16-trimethyl-3-oxo-6,7,8,9,10,11,12,13,14,15,16,17-dodeca-hydro 3H-cyclopenta[a]phenanthren-17-yl ester or any of the examples in WO 02/00679, (R)-3-(2-Hydroxy-2,2-diphenyl-acetoxy)-1-(isoxazol-3-ylcarbamoyl-methyl)-1-azonia-bicyclo-[2.2.2]octane bromide (a quinuclidine) or ((R)-3-((R)-2-cyclohexyl-2-hydroxy-2-phenylacetoxy)-1 -(isoxazol-3-ylcarbamoylmethyl)-1 azoniabicyclo[2.2.2]octane or any of the examples in WO 2004/096800 and WO 2006/048225, salmeterolor formoterol fumarate and mometasone furoate, or (R)-3-(2-Hydroxy-2,2-diphenyl-acetoxy)-1-(isoxazol-3 -ylcarbamoyl-methyl)-1-azonia-bicyclo-[2.2.2]octane bromide (a quinuclidine) or ((R)-3-((R)-2-cyclohexyl-2-hydroxy-2-phenylacetoxy)-1-(isoxazol-3 -ylcarbamoylmethyl)-1-azoniabicyclo[2.2.2]octane, formoterol fumarate and 3-Methyl-thiophene-2-carboxylic acid (6S,9R,10S,11S,13S,16R,17R)-9-chloro -6-fluoro-11-hydroxy-17-methoxycarbonyl-10,13,16-trimethyl-3-oxo-6,7,8,9,10,11,12,13,14,15,16,17-dodeca-hydro -3H-cyclopenta[a]phenanthren-17-yl ester or any of the examples in WO 02/00679, medicaments which have a rapid onset of action and a long duration of action may be prepared. Moreover, using such combination therapies, medicaments which result in a significant improvement in lung function may be prepared. Using the combination therapies of the invention, medicaments which provide improved control of obstructive or inflammatory airways diseases, or a reduction in exacerbations of such diseases, may be prepared. Using compositions of the invention, medicaments which can be used on demand in rescue treatment of obstructive or inflammatory airways diseases, or which reduce or eliminate the need for treatment with short-acting rescue medicaments such as salbutamol or terbutaline, may be prepared; thus medicaments based on compositions of the invention facilitate the treatment of an obstructive or inflammatory airways disease with a single medicament.

Further examples of corticosteroid s that can be used in either of the combinations of (Group I) and (Group II) respectively can be selected from any described in WO 02/100879, WO 2002012265, WO 2002012266, WO 2002088167
WO 03/035668, WO 03/048181, WO 03/062259, WO 03/064445, WO 03/072592, WO 2003042229, WO 2003042230, WO 2003048181,WO 2005005451,WO 2005005452, WO 2005028495, WO 2006072600, WO 2006072599. In particular, the corticosteroids can be selected from 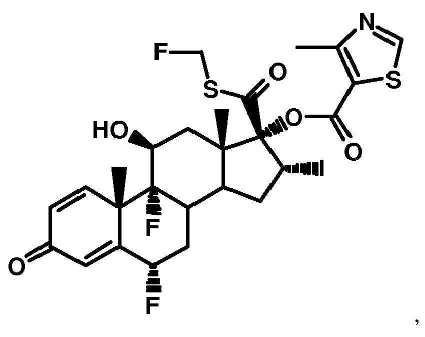 4-Methyl-thiazole-5-carboxylic acid (6S,9R,10S,11S,13S,16R,17R)-6,9-difluoro-17 fluoromethylsulfanylcarbonyl-11-hydroxy-10,13,16-trimethyl-3-oxo-6,7,8,9,10,11,12,13,14,15,16,17-dodecahydro-3H-cyclopenta[a]phenanthren-17-yl ester and 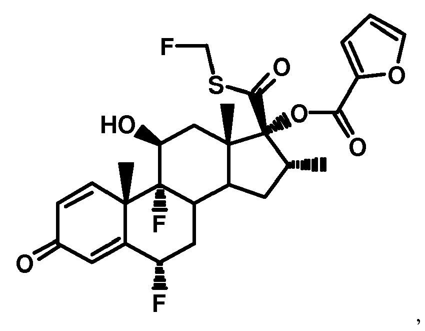 Furan-2-carboxylic acid (6S,9R,10S,11S, 13S, 16R,17R)-6,9-difluoro-17-fluoromethylsulfanylcarbonyl-11-hydroxy-10,13, 16-trimethyl-3-oxo-6,7,8,9, 10,11, 12, 13, 14, 15,16,17-dodecahydro-3H-cyclopenta[a]phenanthren-17-yl ester.

In another aspect, the present invention provides a pharmaceutical composition comprising a mixture of effective amounts of (A), (B) and (C) of either (Group I), (Group II), (Group III), or (Group IV) respectively, as hereinbefore defined, optionally together with at least one pharmaceutically acceptable carrier.

In a nother aspect, the present invention provides a method of treating an inflammatory or obstructive airways disease which comprises administering to a subject in need of such treatment effective amounts of (A), (B) and (C) of either (Group I), (Group II), (Group III), or (Group IV) respectively, as hereinbefore defined.

The invention further provides the use of compounds (A), (B) and (C) of either (Group I), (Group II), (Group III), or (Group IV) respectively, as hereinbefore defined in the preparation of a medicament for combination therapy by simultaneous, sequential or separate administration of either (Group I), (Group II), (Group III), or (Group IV) respectively, in the treatment of an inflammatory or obstructive airways disease.

Another aspect of the invention provides compounds of formula (I) of (Group II) and (Group IV),
where
R¹ and R³ are each independently suitably a C₆-C₁₅-aromatic carbocyclic group or a 4- to 12-membered heterocyclic group having at least one ringheteroatom selected from nitrogen, oxygen and sulphur;
R² is halo or hydroxyl;
R⁴ is C₁-C₈-alkyl substituted by -NHR⁵, -NR⁵-CO-R⁶, -NR⁵-CO-NH-R⁷, -NR⁵-SO₂-R⁸, -CO-NR⁹R¹⁰, -O-CO-NH-R¹², -O-CO-R¹³ or -CO-O-R¹⁴,
or R⁴ is C₃-C₈-alkynyl optionally substituted by a C₃-C₁₅-carbocyclic group, C₆-C₁₅-aromatic carbocyclic group, or a 4- to 12-membered heterocyclic group having at least one ring heteroatom selected from nitrogen, oxygen and sulphur;
R⁵ is hydrogen or C₁-C₄-alkyl;
R⁶ is C₁-C₄-alkyl, C₂-C₈-alkynyl or C₁-C₄-alkoxy in each case optionally substituted by a C₃-C₁₅-carbocyclic group, C₆-C₁₅-aromatic carbocyclic group, or a 4- to 12-membered heterocyclic group having at least one ring heteroatom selected from nitrogen, oxygen and sulphur,
or R⁶ is a C₃-C₁₅-carbocyclic group, C₆-C₁₅-aromatic carbocyclic group, or a 4- to 10-membered heterocyclic group having at least one ring heteroatom selected from nitrogen, oxygen and sulphur;
R⁷ is a C₃-C₁₅-carbocyclic group or C₆-C₁₅-aromatic carbocyclic group;
R⁸ is a C₃-C₁₅-carbocyclic group or C₆-C₁₅-aromatic carbocyclic group;
R⁹ is hydrogen or C₁-C₄-alkyl;
R¹⁰ is C₁-C₄-alkyl optionally substituted by cyano, C₁C₄-alkoxy, a C₃-C₁₅-carbocyclic group, C₆-C₁₅-aromatic carbocyclic group, or by a 4-to 12-membered heterocyclic group having at least one ring heteroatom selected from nitrogen, oxygen and sulphur,
or R¹⁰ is a C₃-C₁₅-carbocyclic group, C₆-C₁₅-aromatic carbocyclic group, or a 4- to 12-membered heterocyclic group having at least one ring heteroatom selected from nitrogen, oxygen and sulphur;
R¹² is a C₃-C₁₅-carbocyclic group or C₆-C₁₅-aromatic carbocyclic;
R¹³ is C₁-C₄-alkyl; and
R¹⁴ is hydrogen, a C₃-C₁₅-carbocyclic group, C₆-C₁₅-aromatic carbocyclic group, or C₁C₄-alkyl optionally substituted by a C₃-C₁₅-carbocylic group or C₆-C₁₅-aromatic carbocyclic group.

According to compounds of formula (I) of (Group II) and (Group IV), R¹ and R³ are each independently a C₃-C₁₅-carbocyclic group, C₆-C₁₅-aromatic carbocyclic group, or a 4-to 12-membered heterocyclic group having at least one ring heteroatom selected from nitrogen, oxygen and sulphur. Preferably, R¹ is a C₆-aromatic carbocyclic group such as phenyl and R³ is preferably either phenyl or a C₆-carbocyclic group such as cyclohexyl.

According to formula (I), R² is suitably hydroxyl.

According to formula (I), R⁴ is suitably C₁-C₈-alkyl substituted by -CO-NR⁹R¹⁰, where R⁹ is suitably hydrogen or C₁-C₄-alkyl. Preferably R⁹ is hydrogen.
R¹⁰ is suitably a 4- to 12-membered heterocyclic group having at least one ring heteroatom selected from nitrogen, oxygen and sulphur. Preferably R¹⁰ is an isoxazole group. More preferably R¹⁰ is a 3-linked isoxazole group.

In one aspect, the present invention provides a medicament comprising, separately or together, the compounds (A), (B) and (C) of either (Group I), (Group II), (Group III), or (Group IV) respectively, for simultaneous, sequential or separate administration in the treatment of an inflammatory or obstructive airways disease.

Compounds of formula (I) in free or salt or solvate form act as muscarinic antagonists, particularly muscarinic M3 receptor antagonists, thereby inhibiting acetylcholine-induced contraction of smooth muscle in e.g. respiratory tract, digestive tract and urinary systems as described in WO 2004/096800 and WO 2006/048225.

Compounds of formula I in free or salt or solvate form may be prepared by using the procedures described in WO 2004/096800 and WO 2006/048225.

Compounds of formula (I) in free form may be converted into salt form, and vice versa, in a conventional manner. The compounds in free or salt form can be obtained in the form of hydrates or solvates containing a solvent used for crystallisation. Compounds of formula I can be recovered from reaction mixtures and purified in a conventional manner. Isomers, such as enantiomers, may be obtained in a conventional manner, e.g. by fractional crystallisation or asymmetric synthesis from correspondingly asymmetrically substituted, e.g. optically active, starting materials.

Pharmaceutically acceptable salts of the compound of formula I may be acid addition salts, including those of inorganic acids, for example hydrohalic acids such as hydrofluoric acid, hydrochloric acid, hydrobromic acid or hydroiodic acid, nitric acid, sulfuric acid, phosphoric acid; and organic acids such as formic acid, acetic acid, propionic acid, butyric acid, benzoic acid, o-hydroxybenzoic acid, p-hydroxybenzoic acid, p-chlorobenzoic acid, diphenylacetic acid, triphenylacetic acid, 1-hydroxynaphthalene-2-carboxylic acid, 3-hydroxynaphthalene-2-carboxylic acid, aliphatic hydroxy acids such as lactic acid, citric acid, tartaric acid or malic acid, dicarboxylic acids such as fumaric acid, maleic acid or succinic acid, and sulfonic acids such as methanesulfonic acid, 4-methylbenzenesulfonic acid or benzenesulfonic acid. These salts may be prepared from compounds of formula I by known salt-forming procedures. Pharmaceutically acceptable solvates are generally hydrates.

Glycopyrronium salts include glycopyrronium bromide, also known as glycopyrrolate, which is known to be an effective antimuscarinic agent. More specifically it inhibits acetyl choline binding to M3 muscarinic receptors thereby inhibiting bronchoconstriction.

Glycopyrrolate is a quaternary ammonium salt. Suitable counter ions are pharmaceutically acceptable counter ions including, for example, fluoride, chloride, bromide, iodide, nitrate, sulfate, phosphate, formate, acetate, trifluoroacetate, propionate, butyrate, lactate, citrate, tartrate, malate, maleate, succinate, benzoate, p-chlorobenzoate, diphenyl-acetate or triphenylacetate, o-hydroxybenzoate, p-hydroxybenzoate, 1-hydroxynaphthalene-2-carboxylate, 3-hydroxynaphthalene-2-carboxylate, methanesulfonate and benzenesulfonate. Its bromide salt, namely 3-[(cyclopentyl-hydroxyphenylacetyl)oxy]-1,1-dimethylpyrrolidinium bromide, has the following structural formula 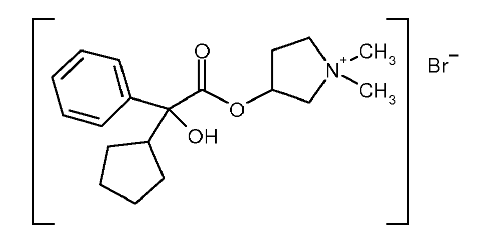 and can be prepared using the procedures described in United States patent US 2956062.

Glycopyrrolate has two stereogenic centres and hence exists in four isomeric forms, namely (3R,2'R)-, (3S,2'R)-, (3R,2'S)-and (3S,2'S)-3-[(cyclopentyl-hydroxyphenylacetyl)oxy]-1,1-dimethylpyrrolidinium bromide, as described in United States patent specifications US 6307060 and US 6,613,795. The contents of these patent specifications are incorporated herein by reference. The present invention embraces using one or more of these isomeric forms, especially the 35,2'R isomer, the 3R,2'R isomer or the 3R,2'S isomer, thus including single enantiomers, mixtures of diastereomers, or racemates, especially (35,2'R/3R,2'S)-3-[(cyclopentyl-hydroxyphenylacetyl)oxy]-1,1-dimethylpyrrolidinium bromide.

Mometasone furoate, (11β,16α)-9,21-dichloro-17-[(2-furanylcarbonyl)oxy]-11-hydroxy-16-methylpregna-1, 4-diene-3,20-dione, alternatively designated 9α,21-dichloro-16α-methyl-1,4-pregnadiene-11β,17α-diol-3,20-dione 17-(2'-furoate), is a topical anti-inflammatory corticosteroid that has the following chemical structure:

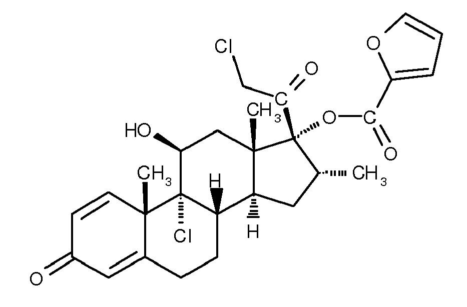

Mometasone furoate and its preparation are described in US 4472393. It use in the treatment of asthma is described in US 5889015. It use in the treatment of other respiratory diseases is described in US 5889015, US 6057307, US 6057581, US 6677322, US 6677323 and US 6365581.

Compounds of formula II are disclosed, together with procedures for their preparation in international patent application WO 02/00679, the contents of which is incorporated herein by reference. These compounds exhibit surprisingly low systemic side effects at therapeutically effective doses and have a long duration of action, with a potential for once-a-day administration.

In one embodiment, T is a heterocyclic aromatic group having a 5-membered heterocyclic ring with one, two or three ring hetero atoms selected from nitrogen, oxygen and sulfur, the heterocyclic ring being unsubstituted or substituted by one or two substituents selected from halogen, C₁-C₄-alkyl, halo-C₁-C₄-alkyl, C₁-C₄-alkoxy, C₁-C₄-alkyl-thio, cyano or hydroxy-C₁-C₄-alkyl and the heterocyclic ring being optionally fused to a benzene ring. Preferred such heterocyclic aromatic groups include those in which the heterocyclic ring has one nitrogen, oxygen or sulfur atom in the ring or one oxygen and one or two nitrogen atoms in the ring, or one sulfur and one or two nitrogen atoms in the ring, especially a pyrrole, furan, thiophene, oxazole, isoxazole, imidazole, pyrazole, furazan, thiazole or thiadiazole ring. Especially preferred heterocyclic aromatic groups are pyrrolyl, furyl and thienyl groups optionally substituted by one or two substituents selected from halogen (particularly chlorine or bromine), C₁-C₄-alkyl (particularly methyl or ethyl), halo-C₁-C₄-alkyl (particularly trifluoro-methyl), Ci-C₄-alkoxy (particularly methoxy), C₁-C₄-alkylthio (particularly methylthio), cyano or hydroxy C₁-C₄-alkyl (particularly hydroxymethyl); isoxazolyl, imidazolyl, pyrazolyl, thiazolyl or thiadiazolyl groups optionally substituted by one or two C₁-C₄-alkyl groups; and benzofuryl, benzothienyl and benzofurazanyl groups.

In another embodiment, T is a heterocyclic aromatic group having a 6-membered heterocyclic ring with one, two or three ring heteroatoms, preferably nitrogen, the heterocyclic ring being unsubstituted or substituted by one or more, preferably one, two or three, substituents selected from halogen, cyano, hydroxyl, C₁-C₄-acyloxy, amino, C₁-C₄-alkyl-amino, di-(C₁-C₄-alkyl)amino, C₁-C₄-alkyl, hydroxy-C₁-C₄-alkyl, halo-C₁-C₄-alkyl, C₁-C₄-alkoxy, or C₁-C₄-alkylthio, and the heterocyclic ring being optionally fused to a benzene ring. Preferred such heterocyclic aromatic groups include those in which the heterocyclic group has one or two nitrogen atoms in the ring, especially a pyridine, pyrimidine, pyrazine or pyridazine ring.

Especially preferred heterocyclic aromatic groups are pyridyl, pyrimidinyl and pyrazinyl groups, optionally substituted by one or two substituents selected from halogen (particularly chlorine) or C₁-C₄-alkyl (especially methyl or n-butyl).

In compounds of formula II, the indicated methyl group in the 16 position of the corticosteroid ring system may be in the alpha or beta conformation. 16-α-methyl compounds are preferred.

Especially preferred compounds of formula II are those where the indicated 16-methyl group has the alpha conformation and T is 5-methyl-2-thienyl, N-methyl-2-pyrrolyl, cyclopropyl, 2-furyl, 3-methyl-2-furyl, 3-methyl-2-thienyl, 5-methyl-3-isoxazolyl, 3,5 climethyl-2-thienyl, 2,5-dimethyl-3-furyl, 4-methyl-2-furyl, 4-(dimethylamino)phenyl, 4-methylphenyl, 4-ethylphenyl, 2-pyridyl, 4 pyrimidyl or 5-methyl-2-pyrazinyl or the indicated 16-methyl group has the beta conformation and R is cyclopropyl.

A particularly preferred compound of formula II is 3-methyl-thiophene-2-carboxylic acid (6S,9R,10S,11S,13S,16R,17R)-9-chloro-6-fluoro-11-hydroxy-17-methoxycarbonyl-10,13,16-trimethyl-3-oxo-6,7,8,9,10,11,12,13,14,15,16,17-dodecahydro-3H-cyclopenta-[a]phenanthren-17-yl ester, which has the formula

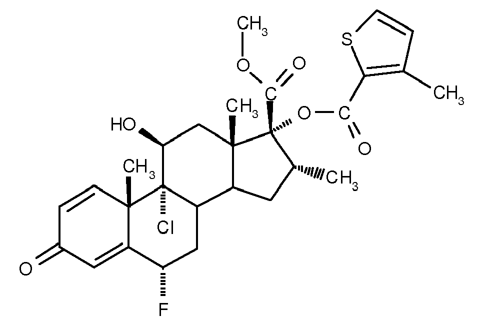

Compounds of formula II in which T contains a basic group are capable of forming acid addition salts, particularly pharmaceutically acceptable acid addition salts. Pharmaceutically acceptable acid addition salts of the compound of formula I include those of inorganic acids, for example, hyd rohalic acids such as hydrofluoric acid, hydrochloric acid, hydrobromic acid or hydroiodic acid, nitric acid, sulfuric acid, phosphoric acid; and organic acids, for example aliphatic monocarboxylic acids such as formic acid, acetic acid, trifluoroacetic add, propionic acid and butyric acid, aliphatic hydroxy acids such as lactic acid, citric acid, tartaric acid or malic acid, dicarboxylic acids such as maleic acid or succinic acid, aromatic carboxylic acids such as benzoic acid, p-chlorobenzoic acid, diphenylacetic acid or triphenylacetic acid, aromatic hydroxy acids such as o-hydroxybenzoic acid, p-hydroxybenzoic acid, 1-hydroxynaphthalene-2-carboxylic acid or 3-hydroxynaphthalene-2-carboxylic acid, and sulfonic acids such as methanesulfonic acid or benzenesulfonic acid. These salts may be prepared from compounds of formula II by known salt-forming procedures.

Administration of the medicament or pharmaceutical composition as hereinbefore described, i.e. with (A), (B) and (C) of either (Group I), (Group II), (Group III), or (Group IV) respectively, in admixture or separate, is preferably by inhalation, i.e. (A), (B) and (C) of either Group I, Group II, Group III, or Group IV respectively, or the mixture thereof are in inhalable form.

The inhalable form of the medicament may be, for example, an atomizable composition such as an aerosol comprising the active ingredients, i.e. (A), (B) and (C) of either (Group I), (Group II), (Group III), or (Group IV) respectively, separately or in admixture, in solution or dispersion in a propellant, or a nebulisable composition comprising a solution or dispersion of the active ingredient in an aqueous, organic or aqueous/organic medium. For example, the inhalable form of the medicament may be an aerosol comprising a mixture of (A), (B) and (C) of either (Group I), (Group II), (Group III), or (Group IV) respectively, in solution or dispersion in a propellant, or a combination of an aerosol containing (A) and (B) of either (Group I), (Group II), (Group III), or (Group IV) respectively, in solution or dispersion in a propellant with an aerosol containing (C) of either (Group I), (Group II), (Group III), or (Group IV) respectively, in solution or dispersion in a propellant. In another example, the inhalable form is a nebulizable composition comprising a dispersion of (A), (B) and (C) of either (Group I), (Group II), (Group III), or (Group IV) respectively, in an aqueous, organic or aqueous/organic medium, or a combination of a dispersion of (A) of either (Group I), (Group II), (Group III), or (Group IV) respectively, in such a medium with a dispersion of (B) of either (Group I), (Group II), (Group III), or (Group IV) respectively, in such a medium and a dispersion of (C)of either (Group I), (Group II), (Group III), or (Group IV) respectively, in such a medium.

An aerosol composition suitable for use as the inhalable form of the medicament may comprise the active ingredient in solution or dispersion in a propellant, which may be chosen from any of the propellants known in the art. Suitable such propellants include hydrocarbons such as n-propane, n-butane or isobutane or mixtures of two or more such hydrocarbons, and halogen-substituted hydrocarbons, for example chlorine and/or fluorine-substituted methanes, ethanes, propanes, butanes, cyclopropanes or cyclobutanes, such as dichlorodifluoromethane (CFC-12), trichlorofluoromethane (CFC-11), 1,2-dichloro -1,1,2,2 -tetrafluoroethane (CFC-114) or, particularly, 1,1,1,2-tetrafluoroethane (HFA-134a), 1,1,1,2,3,3,3 -heptafluoropropane (HFA-227), difluorochloromethane (HCFC-22) or mixtures of two or more such halogen -substituted hydrocarbons.

Where the active ingredient is present in suspension in the propellant, i.e. where it is present in particulate form dispersed in the propellant, the aerosol composition may also contain a lubricant and a surfactant, which may be chosen from those lubricants and surfactants known in the art. Other suitable aerosol compositions include surfactant-free or substantially surfactant-free aerosol compositions. The aerosol composition may contain up to about 5% by weight, for example 0.0001 to 5 % , 0.001 to 5 % , 0.001 to 3 % , 0.001 to 2% , 0.001 to 1 % , 0.001 to 0.1 % , or 0.001 to 0.01 % , but preferably 0.01 to 0.5 % by weight of the active ingredient, based on the weight of the propellant. Where present, the lubricant and surfactant may be in an amount up to 5% and 0.5 % respectively by weight of the aerosol composition. The aerosol composition may also contain a co -solvent such as ethanol in an amount up to 30% by weight of the composition, particularly for administration from a pressurised metered dose inhalation device. The aerosol composition may further contain a bulking agent, for example a sugar such as lactose, sucrose, dextrose, mannitol or sorbitol, in an amount, for example, of up to 20% , usually 0.001 to 1 % , by weight of the composition.

In another embodiment of the invention, the inhalable form of the medicament is a dry powder, i.e. (A), (B) and (C)of either (Group I), (Group II), (Group III), or (Group IV) respectively, are present in a dry powder comprising finely divided (A), (B) and (C) of either (Group I), (Group II), (Group III), or (Group IV) respectively, optionally together with at least one particulate pharmaceutically acceptable carrier, which maybe one or more materials known as pharmaceutically acceptable carriers, preferably chosen from materials known as carriers in dry powder inhalation compositions, for example saccharides, including monosaccharides, disaccharides, polysaccharides and sugar alcohols such as arabinose, glucose, fructose, ribose, mannose, sucrose, trehalose, lactose, maltose, starches, dextran, mannitol or sorbitol. An especially preferred carrier is lactose, for example lactose monohydrate or anhydrous lactose. The dry powder may be contained as unit doses in capsules of, for example, gelatin or plastic, or in blisters (e.g. of aluminium or plastic), for use in a dry powder inhalation device, which may be a single dose or multiple dose device, preferably in dosage units of (A), (B) and/or (C) of either (Group I), (Group II), (Group III), or (Group IV) respectively, together with the carrier in amounts to bring the total weight of powder per capsule or suitable pre-metered dose unit such as blister to from 5 mg to 50 mg. Alternatively, the dry powder may be contained in a reservoir in a multi-dose dry powder inhalation (MDDPI) device adapted to deliver, for example, 1-25 mg of dry powder per actuation.

In the finely divided particulate form of the medicament, and in the aerosol composition where at least one of the active ingredients are present in particulate form, the active ingredient may have an average particle diameter of up to about 10 µm, for example 0.1 to 5 µm, preferably 1 to 5 µm. The particulate carrier, where present, generally has a maximum particle diameter up to 5 00 µm, preferably up to 400 µm, and conveniently has a mean particle diameter of 40 to 300 µm, e.g. 50 to 250 µm. The particle size of the active ingredient, and that of a particulate carrier where present in dry powder compositions, can be reduced to the desired level by conventional methods, for example by grinding in an air-jet mill, ball mill or vibrator mill, sieving, microprecipitation, spray-drying, lyophilisation or controlled crystallisation from conventional solvents or from supercritical media.

The inhalable medicament may be administered using an inhalation device suitable for the inhalable form, such devices being well known in the art. Accordingly, the invention also provides a pharmaceutical product comprising a medicament or pharmaceutical composition as hereinbefore described in inhalable form as hereinbefore described in association with one or more inhalation devices. In a further aspect, the invention provides an inhalation device, or a pack of two or more inhalation devices, containing a medicament or pharmaceutical composition as hereinbefore described in inhalable form as hereinbefore described.

Where the inhalable form of the active ingredient is an aerosol composition, the inhalation device may be an aerosol vial provided with a valve adapted to deliver a metered dose, such as 10 to 100 µl, e.g. 25 to 50 µl, of the composition, i.e. a device known as a metered dose inhaler. Suitable such aerosol vials and procedures for containing within them aerosol compositions under pressure are well known to those skilled in the art of inhalation therapy. For example, an aerosol composition may be administered from a coated can, for example as described in EP-A-0642992.
Where the inhalable form of the active ingredient is a nebulizable aqueous, organic or aqueous/organic dispersion, the inhalation device may be a known nebulizer, for example a conventional pneumatic nebulizer such as an airjet nebulizer, or an ultrasonic nebulizer, which may contain, for example, from 1 to 50 ml, commonly 1 to 10 ml, of the dispersion; or a handheld nebulizer, sometimes referred to as a soft mist or soft spray inhaler, for example an electronically controlled device such as an AERx (Aradigm, US) or Aerodose (Aerogen), or a mechanical device such as a RESPIMAT (Boehringer Ingelheim) nebulizer which allows much smaller nebulised volumes, e.g. 10 to 100 µl, than conventional nebulisers.

Where the inhalable form of the active ingredient is the finely divided particulate form, the inhalation device may be, for example, a dry powder inhalation device adapted to deliver dry powder from a capsule or blister containing a dry powder comprising a dosage unit of (A) and/or (B) of either (Group I), (Group II), (Group III), or (Group IV), respectively, or a multi-dose dry powder inhalation (MDDPI) device adapted to deliver, for example, 3-25 mg of dry powder comprising a dosage unit of (A) and/or (B) of either (Group I), (Group II), (Group III), or (Group IV) respectively, per actuation. The dry powder composition preferably contains a diluent or carrier, such as lactose, and a compound that helps to protect against product performance deterioration due to moisture and / or improve physical properties (such as flow, dispersion) of the dry powder, e.g. magnesium stearate, typically 0.05-2.0% . Suitable such dry powder inhalation devices are well known. For example, a suitable device for delivery of dry powder in encapsulated form is that described in US 3991761, while suitable MDD PI devices include those described in WO 97/20589, WO 97/30743 and WO 05/37353.

The medicament of the invention is preferably a pharmaceutical composition comprising a mixture of (A) as hereinbefore defined, (B) as hereinbefore defined, and (C) as hereinbefore defined of either (Group I), (Group II), (Group III), or (Group IV) respectively, preferably together with at least one pharmaceutically acceptable carrier as hereinbefore described.

A suitable daily dose of the compound (A) of either (Group I), (Group II), (Group III), or (Group IV) respectively, for inhalation may be from 20µg to 2000 µg, for example from 20 to 1500 µg, from 20 to 1000 µg, preferably from 50 to 800 µg, e.g. from 100 to 600 µg or from 100 to 500 µg.

A suitable daily dose of the compound (B) of either (Group I), (Group II), (Group III), or (Group IV) respectively, for inhalation may be from 10µg to 2000 µg, preferably from 20 to 1000 µg, and especially from 20 to 800 µg, e.g. from 100 to 500 µg.

A suitable daily dose of the compound (C), of either (Group I), (Group II), (Group III), or (Group IV) respectively, for inhalation may be from 50 to 2000 µg, for example from 100 to 2000 µg, from 100 to 1600 µg, from 100 to 1000 µg, or from 100 to 800 µg, preferably from 200 to 500 µg, for instance from 200 to 400 µg.

A suitable unit dose of the compound (A), of either (Group I), (Group II), (Group III), or (Group IV) respectively, for inhalation may be from 10µg to 2000 µg, preferably from 20 to 1000 µg, and especially from 20 to 800 µg, e.g. from 100 to 500 µg.

A suitable unit dose of the compound (B), of either (Group I), (Group II), (Group III), or (Group IV) respectively, for inhalation may be from 20 µg to 2000 µg, for example from 20 to 1500 µg, from 20 to 1000 µg, preferably from 50 to 800 µg, e.g. from 100 to 600 µg or from 100 to 500 µg.

A suitable unit dose of the compound (C), of either (Group I), (Group II), (Group III), or (Group IV) respectively, for inhalation may be from 50 to 2000 µg, for example from 100 to 2000 µg, from 100 to 1 600 µg, from 100 to 1000 µg, or from 100 to 800 µg, preferably from 200 to 500 µg, for instance from 200 to 400 µg.

These unit doses may be administered once or twice daily in accordance with the daily doses mentioned hereinbefore. A single dose is preferred as this is convenient for the patient and encourages compliance. The precise doses of (A), (B) and (C)of either (Group I), (Group II), (Group III), or (Group IV) respectivelyused will of course depend on the condition to be treated, the patient, the formulation and the efficiency of the inhalation device.

In one preferred embodiment of the invention, the medicament of the invention is a pharmaceutical composition which is a dry powder in a capsule containing unit doses of (A), (B) and (C) of either (Group I), (Group II), (Group III), or (Group IV) respectively, for example for inhalation from a single capsule inhaler, the capsule suitably containing a unit dose of (A) e.g. as hereinbefore described, a unit dose of (B), e.g. as herein before described, and a unit dose of (C), e.g. as hereinbefore described, of either (Group I), (Group II), (Group III), or (Group IV) respectively, together with a pharmaceutically acceptable carrier as hereinbefore described in an amount to bring the total weight of dry powder per capsule to between 5 mg and 50 mg, for example 5 mg, 10 mg, 15 mg, 20 mg, 25 mg, 30 mg, 35 mg, 40 mg, 45 mg or 50 mg.

In another preferred embodiment of the invention, the medicament of the invention is a pharmaceutical composition which is a dry powder for administration from a reservoir of a multi-dose dry powder inhaler adapted to deliver, for example, 3 mg to 25 mg of powder containing a unit dose of (A), (B) and (C) of either (Group I), (Group II), (Group III), or (Group IV) respectively, per actuation.

In a further preferred embodiment of the invention, the medicament of the invention is a pharmaceutical composition which is an aerosol comprising (A), (B) and (C) of either (Group I), (Group II), (Group III), or (Group IV)respectively, as hereinbefore described in a propellant as hereinbefore described, optionally together with a surfactant and/or a bulking agent and/or a co-solvent such as ethanol as hereinbefore described, for administration from a metered dose inhaler adapted to deliver an amount of aerosol containing a unit dose of (A), a unit dose of (B), a unit dose of (C), of either (Group I), (Group II), (Group III), or (Group IV) respectively, or a known fraction of a unit dose of (A), a known fraction of a unit dose of (B), and a known fraction of a unit dose of (C) per actuation of either (Group I), (Group II), (Group III), or (Group IV) respectively. Thus if, for example, the inhaler delivers half of the unit doses of (A), (B) and (C) of either (Group I), (Group II), (Group III), or (Group IV), respectively per actuation, the unit doses can be administered by two actuations of the inhaler.

In accordance with the above, the invention also provides a pharmaceutical kit comprising (A), (B) and (C) of either (Group I), (Group II), (Group III), or (Group IV) respectively, as hereinbefore defined in separate unit dosage forms, said forms being suitable for administration of (A), (B) and (C) of either (Group I), (Group II), (Group III), or (Group IV) respectively, in effective amounts. Such a kit suitably further comprises one or more inhalation devices for administration of (A), (B) and (C) of either (Group I), (Group II), (Group III), or (Group IV) respectively. For example, the kit may comprise one or more dry powder inhalation devices adapted to deliver dry powder from a capsule, together with capsules containing a dry powder comprising a dosage unit of (A), capsules containing a dry powder comprising a dosage unit of (B) and capsules containing a dry powder comprising a dosage unit of (C) of either (Group I), (Group II), (Group III), or (Group IV) respectively. In another example, the kit may comprise a multi-dose dry powder inhalation device containing in the reservoir thereof a dry powder comprising (A), a multi-do se dry powder inhalation device containing in the reservoir thereof a dry powder comprising (B) and a multi-dose dry powder inhalation device containing in the reservoir thereof a dry powder comprising (C)of either (Group I), (Group II), (Group III), or (Group IV) respectively. In another example, the kit may comprise a multi-dose dry powder inhalation device containing in the reservoir thereof a dry powder comprising (B) and a multi-dose dry powder inhalation device containing in the reservoir thereof a dry powder comprising a mixture of (A) and (C) of either (Group I), (Group II), (Group III), or (Group IV) respectively. In a yet further example, the kit may comprise a metered dose inhaler containing an aerosol comprising (A) in a propellant, a metered dose inhaler containing an aerosol comprising (B) in a propellant, and a metered dose inhaler containing an aerosol comprising (C) in a propellant of either (Group I), (Group II), (Group III), or (Group IV) respectively.

Medicaments of the invention are advantageous in the treatment of inflammatory or obstructive airways disease, exhibiting highly effective bronchodilatory and anti-inflammatory properties. For instance, it is possible using the combination therapy of the invention to reduce the dosages of corticosteroid required for a given therapeutic effect compared with those required using treatment with a corticosteroid alone, thereby minimising possibly undesirable side effects. In particular, these combinations, particularly where (A), (B) and (C) of either (Group I), (Group II), (Group III), or (Group IV) respectively, are in the same composition, facilitate achievement of a high anti-inflammatory effect, such that the amount of corticosteroid needed for a given anti-inflammatory effect may be reduced when used in admixture with (A) and (B) of either (Group I), (Group II), (Group III), or (Group IV) respectively, thereby reducing the risk of undesirable side effects from the repeated exposure to the steroid involved in the treatment of inflammatory orobstructive airways diseases. Furthermore, using the combinations of the invention, particularly using compositions containing (A), (B) and (C) of either (Group I), (Group II), (Group III), or (Group IV) respectively, medicaments which have a rapid onset of action and a long duration of action may be prepared. Moreover, using such combination therapy, medicaments which result in a significant improvement in lung function may be prepared. In another aspect, using the combination therapy of the invention, medicaments which provide effective control of obstructive or inflammatory airways diseases, or a reduction in exacerbations of such diseases, may be prepared. In a further aspect, using compositions of the invention containing (A), (B) and (C) of either (Group I), (Group II), (Group III), or (Group IV) respectively, medicaments which reduce or eliminate the need for treatment with short-acting rescue medicaments such as salbutamol or terbutaline, may be prepared; thus compositions of the invention containing (A), (B) and (C) of either (Group I), (Group II), (Group III), or (Group IV) respectively, facilitate the treatment of an obstructive or inflammatory airways disease with a single medicament.

Treatment of inflammatory or obstructive airways diseases in accordance with the invention may be symptomatic or prophylactic treatment. Inflammatory or obstructive airways diseases to which the present invention is applicable include asthma of whatever type or genesis including both intrinsic (non-allergic) asthma and extrinsic (allergic) asthma, mild asthma, moderate asthma, severe asthma, bronchitic asthma, exercise-induced asthma, occupational asthma and asthma induced following bacterial infection. Treatment of asthma is also to be understood as embracing treatment of subjects, e.g. of less than 4 or 5 years of age, exhibiting wheezing symptoms and diagnosed or diagnosable as "wheezy infants", an established patient category of major medical concern and now often identified as incipient or early-phase asthmatics. (For convenience this particular asthmatic condition is referred to as "wheezy-infant syndrome".)

Prophylactic efficacy in the treatment of asthma will be evidenced by reduced frequency or severity of symptomatic attack, e.g. of acute asthmatic or bronchoconstrictor attack, improvement in lung function or improved airways hyperreactivity. It may further be evidenced by reduced requirement for other, symptomatic therapy, i.e. therapy for or intended to restrict or abort symptomatic attack when it occurs, for example anti-inflammatory (e.g. corticosteroid) or bronchodilatory. Prophylactic benefit in asthma may in particular be apparent in subjects prone to "morning dipping". "Morning dipping" is a recognised asthmatic syndrome, common to a substantial percentage of asthmatics and characterised by asthma attack, e.g. between the hours of about 4 to 6 am, i.e. at a time normally substantially distant form any previously administered symptomatic asthma therapy.

Other inflammatory or obstructive airways diseases and conditions to which the present invention is applicable include acute lung injury (ALI), adult or acute respiratory distress syndrome (ARDS), chronic obstructive pulmonary, airways or lung disease (COPD, CO AD or COLD), including chronic bronchitis and emphysema, bronchiectasis and exacerbation of airways hyperreactivity consequent to other drug therapy, in particular other inhaled drug therapy. Further inflammatory or obstructive airways diseases to which the present invention is applicable include pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tobacosis and byssinosis.

The invention is illustrated by the following Examples.

### EXAMPLES

### Compound A1

(R)-3-(2-Hydroxy-2,2-diphenyl-acetoxy)-1-(isoxazol-3-ylcarbamoyl-methyl)-1-azonia-bicyclo-[2.2.2]octane bromide (a quinuclidine) or ((R)-3-((R)-2-cyclohexyl-2-hydroxy-2-phenylacetoxy)-1-(isoxazol-3-ylcarbamoylmethyl)-1-azoniabicyclo[2.2.2]octane as described or is prepared using the procedures described in WO 2004/096800 and WO 2006/048225.

### Compound B1

Formoterol fumarate dihydrate as described in U.S. Pat. No. 3,994,974 or U.S. Pat. No. 5,684,199.

### Compound C1

### Mometasone furoate

This compound is prepared using the procedures described in US 4472393.

### Examples 1-60

Gelatin capsules suitable for use in a capsule inhaler such as that described in US 3991761 and EP 1270034 are prepared, each capsule containing a dry powder obtained by mixing Compound A1, Compound B1 and Compound C1, which have been milled to a mean particle diameter of 1 to 5 µm and lactose monohydrate having a particle diameter below 300 µm, the amounts being as shown in the Table 1 below:

**TABLE 1**

| Example | Compound A1 (Parts) | Compound B1 (Parts) | Compound C1 (Parts) | Lactose (Parts) |
|---|---|---|---|---|
| 1 | 50 | 10 | 20 | 19930 |
| 2 | 50 | 10 | 40 | 199100 |
| 3 | 50 | 10 | 80 | 19860 |
| 4 | 50 | 10 | 100 | 19840 |
| 5 | 50 | 10 | 120 | 19820 |
| 6 | 50 | 10 | 140 | 19800 |
| 7 | 50 | 10 | 160 | 19780 |
| 8 | 50 | 10 | 180 | 19760 |
| 9 | 50 | 10 | 200 | 19740 |
| 10 | 50 | 10 | 220 | 19720 |
| 11 | 50 | 10 | 240 | 19700 |
| 12 | 50 | 10 | 300 | 19640 |
| 13 | 50 | 10 | 500 | 19440 |
| 14 | 50 | 10 | 1000 | 18940 |
| 15 | 50 | 10 | 20 | 24920 |
| 16 | 50 | 10 | 40 | 24900 |
| 17 | 50 | 10 | 80 | 24860 |
| 18 | 50 | 10 | 100 | 24840 |
| 19 | 50 | 10 | 120 | 24820 |
| 20 | 50 | 10 | 140 | 24800 |
| 21 | 50 | 10 | 160 | 24780 |
| 22 | 50 | 10 | 180 | 24760 |
| 23 | 50 | 10 | 200 | 24740 |
| 24 | 50 | 10 | 220 | 24720 |
| 25 | 50 | 10 | 240 | 24700 |
| 26 | 50 | 10 | 300 | 24640 |
| 27 | 50 | 10 | 500 | 24440 |
| 28 | 50 | 10 | 1000 | 23940 |
| 29 | 100 | 10 | 20 | 14870 |
| 30 | 100 | 10 | 40 | 14850 |
| 31 | 100 | 10 | 80 | 14810 |
| 32 | 100 | 10 | 100 | 14790 |
| 33 | 100 | 10 | 120 | 14770 |
| 34 | 100 | 10 | 140 | 14750 |
| 35 | 100 | 10 | 160 | 14730 |
| 36 | 100 | 10 | 180 | 14710 |
| 37 | 100 | 10 | 200 | 14690 |
| 38 | 100 | 10 | 220 | 14670 |
| 39 | 100 | 10 | 240 | 14650 |
| 40 | 100 | 10 | 300 | 14590 |
| 41 | 100 | 10 | 500 | 14390 |
| 42 | 100 | 10 | 1000 | 13890 |
| 43 | 100 | 10 | 20 | 24870 |
| 44 | 100 | 10 | 40 | 24850 |
| 45 | 100 | 10 | 80 | 24890 |
| 46 | 100 | 10 | 100 | 24790 |
| 47 | 100 | 10 | 120 | 24770 |
| 48 | 100 | 10 | 140 | 24750 |
| 49 | 100 | 10 | 160 | 24730 |
| 50 | 100 | 10 | 180 | 24710 |
| 51 | 100 | 10 | 200 | 24690 |
| 52 | 100 | 10 | 220 | 24670 |
| 53 | 100 | 10 | 240 | 24650 |
| 54 | 100 | 10 | 300 | 24590 |
| 55 | 100 | 10 | 500 | 24390 |
| 56 | 100 | 10 | 1000 | 23890 |

### Examples 57-98

A dry powder suitable for delivery from the reservoir of the multi-dose dry powder inhaler described in WO 97/20589 is prepared by mixing Compound A1, Compound B1 and Compound C1, which have been milled to a mean particle diameter of 1-5 µm and lactose monohydrate having a particle diameter below 300 µm, the amounts being as shown in the Table 2 below:

**TABLE 2**

| Example | Compound A1 (Parts) | Compound B1 (Parts) | Compound C1 (Parts) | Lactose (Parts) |
|---|---|---|---|---|
| 57 | 50 | 10 | 20 | 4920 |
| 58 | 50 | 10 | 40 | 4900 |
| 59 | 50 | 10 | 80 | 4860 |
| 60 | 50 | 10 | 100 | 4840 |
| 61 | 50 | 10 | 120 | 4820 |
| 62 | 50 | 10 | 140 | 4800 |
| 63 | 50 | 10 | 160 | 4780 |
| 64 | 50 | 10 | 180 | 4760 |
| 65 | 50 | 10 | 200 | 4740 |
| 66 | 50 | 10 | 220 | 4720 |
| 67 | 50 | 10 | 240 | 4700 |
| 68 | 50 | 10 | 300 | 4640 |
| 69 | 50 | 10 | 500 | 4440 |
| 70 | 50 | 10 | 1000 | 3940 |
| 71 | 100 | 10 | 20 | 9870 |
| 72 | 100 | 10 | 40 | 9850 |
| 73 | 100 | 10 | 80 | 9810 |
| 74 | 100 | 10 | 100 | 9790 |
| 75 | 100 | 10 | 120 | 9770 |
| 76 | 100 | 10 | 140 | 9750 |
| 77 | 100 | 10 | 160 | 9730 |
| 78 | 100 | 10 | 180 | 9710 |
| 79 | 100 | 10 | 200 | 9690 |
| 80 | 100 | 10 | 220 | 9670 |
| 81 | 100 | 10 | 240 | 9650 |
| 82 | 100 | 10 | 300 | 9590 |
| 83 | 100 | 10 | 500 | 9390 |
| 84 | 100 | 10 | 1000 | 8890 |
| 85 | 150 | 10 | 20 | 14820 |
| 86 | 150 | 10 | 40 | 14800 |
| 87 | 150 | 10 | 80 | 14760 |
| 88 | 150 | 10 | 100 | 14740 |
| 89 | 150 | 10 | 120 | 14720 |
| 90 | 150 | 10 | 140 | 14700 |
| 91 | 150 | 10 | 160 | 14680 |
| 92 | 150 | 10 | 180 | 14660 |
| 93 | 150 | 10 | 200 | 14640 |
| 94 | 150 | 10 | 220 | 14620 |
| 95 | 150 | 10 | 240 | 14600 |
| 96 | 150 | 10 | 300 | 14540 |
| 97 | 150 | 10 | 500 | 14340 |
| 98 | 150 | 10 | 1000 | 13840 |

### Examples 99-126

A dry powder suitable for delivery from the pre-metered dose unit or blister of the multi-dose dry powder inhaler described in WO 05/37353 is prepared by mixing Compound A1, Compound B1 and Compound C1, which have been milled to a mean particle diameter of 1-5 µm and lactose monohydrate having a particle diameter below 300 µm, the amounts being as shown in the Table 3 below:

**TABLE 3**

| Example | Compound A1 (Parts) | Compound B1 (Parts) | Compound C1 (Parts) | Lactose (Parts) |
|---|---|---|---|---|
| 99 | 100 | 10 | 50 | 9840 |
| 100 | 100 | 10 | 100 | 9790 |
| 101 | 100 | 10 | 150 | 9740 |
| 102 | 100 | 10 | 200 | 9690 |
| 103 | 100 | 10 | 250 | 9640 |
| 104 | 100 | 10 | 300 | 9590 |
| 105 | 100 | 10 | 350 | 9540 |
| 106 | 100 | 20 | 50 | 9830 |
| 107 | 100 | 20 | 100 | 9780 |
| 108 | 100 | 20 | 150 | 9730 |
| 109 | 100 | 20 | 200 | 9680 |
| 110 | 100 | 20 | 250 | 9630 |
| 111 | 100 | 20 | 300 | 9580 |
| 112 | 100 | 20 | 350 | 9530 |
| 113 | 150 | 10 | 50 | 14790 |
| 114 | 150 | 10 | 100 | 14740 |
| 115 | 150 | 10 | 200 | 14640 |
| 116 | 150 | 10 | 400 | 14440 |
| 117 | 150 | 10 | 600 | 14240 |
| 118 | 150 | 10 | 800 | 14040 |
| 119 | 150 | 10 | 1000 | 13840 |
| 120 | 150 | 20 | 50 | 14780 |
| 121 | 150 | 20 | 100 | 14730 |
| 122 | 150 | 20 | 200 | 14630 |
| 123 | 150 | 20 | 400 | 14430 |
| 124 | 150 | 20 | 600 | 14230 |
| 125 | 150 | 20 | 800 | 14030 |
| 126 | 150 | 20 | 1000 | 13830 |

### Examples 127-171

A dry powder suitable for delivery from the reservoir of the multi-dose dry powder inhaler described in WO 97/20589 is prepared by mixing Compound A1, Compound B1 and Compound C1, which have been milled to a mean particle diameter of 1-5 µm and lactose monohydrate having a particle diameter below 300 µm, the amounts being as shown in the Table 2 but also containing 0.5% magnesium stearate by weight.

### Examples 172-201

A dry powder suitable for delivery from the reservoir of the multi-dose inhaler described in W0 97/205 89 is prepared by mixing Compound A1, Compound B1 and Compound C1, which have been milled to a mean particle diameter of 1-5 µm and lactose monohydrate having a particle diameter below 300 µm, the amounts being as shown in the Table 3 but also containing 1 % magnesium stearate by weight.

### Examples 202-210

Aero sol formulations are prepared by dispensing micronised active ingredients, Compound A1, Compound B1 and Compound C1, and if required, lactose as bulking agent into a vial, sealing the vial with a metering valve, injecting the premixed ethanol/propellant and optional surfactant into the vial through the valve and subjecting the vial to ultrasonic energy to disperse the solid particles. The components and amounts used are shown in Table 4 below, where OA means oleic acid:

**TABLE 4**

| Ex. | Cpd.A1 (Parts) | Cpd.B1 (Parts) | Cpd.C1 (Parts) | HFA134a (Parts) | HFA227 (Parts) | Ethanol (Parts) | OA (Parts) | Lactose (Parts) |
|---|---|---|---|---|---|---|---|---|
| 202 | 50 | 2 | 100 | 36500 | 60750 | 2500 | - | 70 |
| 203 | 50 | 2 | 100 | 97000 | - | 2500 | - | 90 |
| 204 | 50 | 2 | 100 | 30500 | 67000 | 2500 | 0.5 | 100 |
| 205 | 20 | 2 | 100 | 98000 | - | 2500 | 1 | - |
| 206 | 20 | 2 | 100 | - | 98000 | 2000 | 1 | - |
| 207 | 20 | 2 | 100 | 30000 | 67000 | 2250 | 0.2 | 90 |
| 208 | 20 | 2 | 100 | 30000 | 60000 | 2000 | 0.8 | - |
| 209 | 20 | 2 | 100 | 30000 | 67000 | 2250 | 0.2 | 90 |
| 210 | 20 | 2 | 50 | - | 98000 | 2000 | 1 | - |

## Claims

1. A medicament comprising, separately or together
(A) a glycopyrronium salt;
(B) a beta-2 adrenoceptor agonist selected from salmeterol and formoterol, or pharmaceutically acceptable salts thereof; and
(C) a corticosteroid selected from fluticasone propionate, 4-Methyl-thiazole-5-carboxylic acid (6S,9R,10S,11S,13S,16R,17R)-6,9-difluoro-17fluoromethylsulfanylcarbonyl-11-hydroxy-10,13,16-trimethyl-3-oxo-6,7,8,9,10,11,12,13,14,15,16,17-dodecahydro-3H-cyclopenta[a]phenanthren-17-yl ester, Furan-2-carboxylic acid (6S,9R,10S,11S,13S,16R,17R)-6,9-difluoro-17-fluoromethylsulfanylcarbonyl-11-hydroxy-10,13,16-trimethyl-3-oxo-6,7,8,9,10,11,12,13,14,15,16,17-dodecahydro-3H-cyclopenta[a]phenanthren-17-yl ester, and budesonide;
for simultaneous, sequential or separate administration in the treatment of an inflammatory or obstructive airways disease.

2. A medicament comprising, separately or together
(A) compounds of formula I 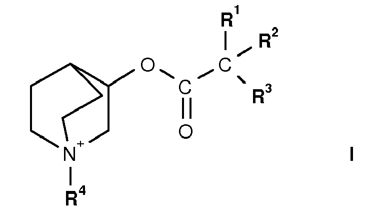 in salt or zwitterionic form wherein
R¹ and R³ are each independently a C₃-C₁₅-carbocyclic group, C₆-C₁₅-aromatic carbocyclic group, or a 4- to 12-membered heterocyclic group having at least one ring heteroatom selected from nitrogen, oxygen and sulphur,
or -CR¹R²R³ together form a group of formula 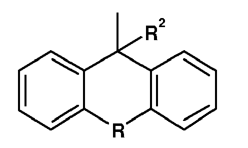 where R is a bond, -O-, -S- , -CH2-, -CH=CH-, -CH₂-CH₂-, amino or -N(CH₃)-;
R² is h ydrogen, halo, hydroxy, C₁-C₈-alkoxy or C₁-C₈-alkyl optionally substituted by hydroxy; R⁴ is C₁-C₈-alkyl substituted by NHR⁵, -NR⁵-CO-R⁶, -NR⁵-CO-NH-R⁷, -NR⁵-SO₂-R⁸ , -CO-NR⁹R¹⁰, -OR¹¹, -O-CO-NHR¹², -O-CO-R¹³ or -CO-O-R¹⁴,
or R⁴ is C₃-C₁₀-alkynyl optionally substituted by a C₃-C₁₅-carbocyclic group, C₆-C₁₅-aromatic carbocyclic group, or a 4- to 12-membered heterocyclic group having at least one ring heteroatom selected from nitrogen, oxygen and sulphur;
R⁵ is hydrogen or C₁C₈-alkyl;
R⁶ is C₁-C₈-alkyl, C₂-C₈-alkenyl, C₂-C₁₀-alkynyl or C₁C₈-alkoxy in each case optionally substituted by a C₃-C₁₅-carbocyclic group, C₆-C₁₅-aromatic carbocyclic group, or a 4- to 12-membered heterocyclic group having at least one ring heteroatom selected from nitrogen, oxygen and sulphur,
or R⁶ is a C₃-C₁₅ is-carbocyclic group, C₆-C₁₅-aromatic carbocyclic group, or a 4- to 12-membered heterocyclic group having at least one ring heteroatom selected from nitrogen, oxygen and sulphur;
R⁷ is a C₃-C₁₅-carbocyclic group or C₆-C₁₅-aromatic carbocyclic group;
R⁸ is a C₃-C₁₅-carbocyclic group or C₆-C₁₅-aromatic carbocyclic group;
R⁹ is hydrogen or C₁C₈-alkyl;
R¹⁰ is hydrogen, C₁-C₈-alkyl optionally substituted by cyano, amino, nitro, carboxy, C₁-C₈-alkoxy, a C₃-C₁₅-carbocyclic group, C₆-C₁₅-aromatic carbocyclic group, or by a 4- to 12-membered heterocyclic group having at least one ring heteroatom selected from nitrogen, oxygen and sulphur,
or R¹⁰ is a C₃-C₁₅-carbocyclic group, C₆-C₁₅-aromatic carbocyclic group, or a 4- to 12-membered heterocyclic group having at least one ring heteroatom selected from nitrogen, oxygen and sulphur;
R¹¹ is hydrogen, C₁-C₈-alkyl, C₁-C₈-alkyl-C₁-C₈-alkoxy or C₁-C₈-alkyl-O-R¹⁵;
R¹² is a C₃-C₁₅-carbocyclic group or C₆-C₁₅-aromatic carbocyclic group;
R¹³ is C₁-C₈-alkyl or a C₃-C₁₅-carbocyclic group or C₆-C₁₅-aromatic carbocyclic group;
R¹⁴ is hydrogen, a C₃-C₁₅-carbocyclic group, C₆-C₁₅-aromatic carbocyclic group, C₁-C₈-alkenyl, or C₁-C₈- alkyl optionally substituted by a C₃-C₁₅-carbocyclic group or C₆-C₁₅-aromatic carbocyclic group;
R¹⁵ is a C₃-C₁₅-carbocyclic group or C₆-C₁₅-aromatic carbocyclic group; and
where each C₃-C₁₅-carbocyclic group is optionally substituted by halo (e.g. fluoro, chloro or bromo), cyano, hydroxy, amino, nitro, carboxy, C₁-C₈-alkyl (e.g. methyl or ethyl), halo-C₁-C₈-alkyl, C₁-C₈-alkoxy, C₁-C₈-alkylcarbonyl, C₁-C₈-alkylsulfonyl, -SO₂NH₂, a C₃-C₁₅-carbocyclic group and a 4- to 12-membered heterocyclic group having at least one ring heteroatom selected from nitrogen, oxygen and sulphur and each C₆-C₁₅-aromatic carbocyclic group is optionally substituted by halo (e.g. fluoro, chloro or bromo), cyano, hydroxy, amino, nitro, carboxy, C₁-C₈-alkyl (e.g. methyl or ethyl), halo-C₁-C₈-alkyl, C₁-C₈-alkoxy, C₁C₈-alkylcarbonyl, C₁-C₈-alkylsulfonyl, -SO₂NH₂, a C₃-C₁₅-carbocyclic group and a 4-to 12-membered heterocyclic group having at least one ring heteroatom selected from nitrogen, oxygen and sulphur;
(B) a beta-2 adrenoceptor agonist selected from salmeterol and formoterol, or pharmaceutically acceptable salts thereof; and
(C) a corticosteroid selected from fluticasone propionate, 4-Methyl-thiazole-5-carboxylic acid (6S,9R,10S,11S,13S,16R,17R)-6,9-difluoro-17fluoromethylsulfanylcarbonyl-11-hydroxy-10,13,16-trimethyl-3-oxo-6,7,8,9,10,11,12,13,14,15,16,17-dodecahydro-3H-cyclopenta[a]phenanthren-17-yl ester,Furan-2-carboxylic acid (6S,9R,10S,11S,13S,16R,17R)-6,9-difluoro-17-fluoromethylsulfanylcarbonyl-11-hydroxy-10,13,16-trimethyl-3-oxo-6,7,8,9,10,11,12,13,14,15,16,17-dodecahydro-3H-cyclopenta[a]phenanthren-17-yl ester, and budesonide;
for simultaneous, sequential or separate administration in the treatment of an inflammatory or obstructive airways disease.

3. A medicament comprising, separately or together
(A) a glycopyrronium salt;
(B) a beta-2 adrenoceptor agonist selected from salmeterol and formoterol, or pharmaceutically acceptable salts thereof; and
(C) a corticosteroid selected from mometasone furoate and a compound of formula II 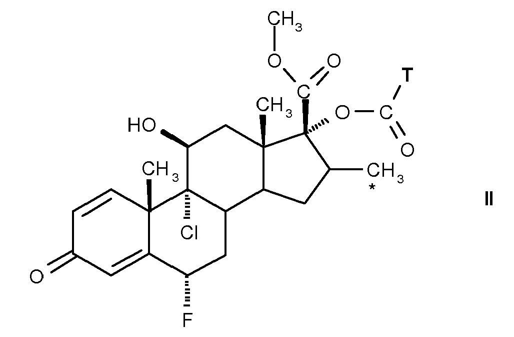 where T is a monovalent cyclic organic group having from 3 to 15 atoms in the ring system;
for simultaneous, sequential or separate administration in the treatment of an inflammatory or obstructive airways disease.

4. A medicament comprising, separately or together
(A) compounds of formula I 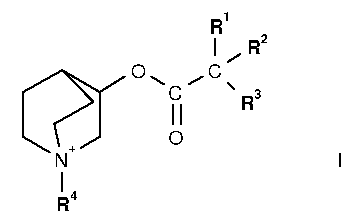 in salt or zwitterionic form wherein
R¹ and R³ are each independently a C₃-C₁₅-carbocyclic group, C₆-C₁₅-aromatic carbocyclic group, or a 4- to 12 -membered heterocyclic group having at least one ring heteroatom selected from nitrogen, oxygen and sulphur,
or -CR¹R²R³ together form a group of formula 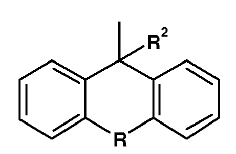 where R is a bond, -O-, -S- , -CH2-, -CH=CH-, -CH₂-CH₂-, amino or -N(CH₃)-;
R² is hydrogen, halo, hydroxy, C₁-C₈-alkoxy or C₁-C₈-alkyl optionally substituted by hydroxy; R⁴ is C₁C₈-alkyl substituted by NHR⁵, -NR⁵-CO-R⁶, -NR⁵-CO-NH-R⁷, -NR⁵-SO₂-R⁸ -CO-NR⁹R¹⁰, -OR¹¹, -O-CO-NHR¹², -O-CO-R¹³ or -CO-O-R¹⁴,
or R⁴is C₃-C₁₀-alkynyl optionally substituted by a C₃-C₁₅-carbocyclic group, C₆-C₁₅-aromatic carbocyclic group, or a 4- to 12-membered heterocyclic group having at least one ring heteroatom selected from nitrogen, oxygen and sulphur;
R⁵ is hydrogen or C₁C₈-alkyl;
R⁶ is C₁-C₈-alkyl, C₂-C8-alkenyl, C₂C₁₀-alkynyl or C₁-C₈-alkoxy in each case optionally substituted by a C₃-C₁₅-carbocyclic group, C₆-C₁₅-aromatic carbocyclic group, or a 4- to 12-membered heterocyclic group having at least one ring heteroatom selected from nitrogen, oxygen and sulphur,
or R⁶ is a C₃-C₁₅-carbocyclic group, C₆-C₁₅-aromatic carbocyclic group, or a 4- to 12-membered heterocyclic group having at least one ring heteroatom selected from nitrogen, oxygen and sulphur;
R⁷ is a C₃-C₁₅-carbocyclic group or C₆-C₁₅-aromatic carbocyclic group;
R⁸ is a C₃-C₁₅-carbocyclic group or C₆-C₁₅-aromatic carbocyclic group;
R⁹ is hydrogen or C₁-C₈-alkyl;
R¹⁰ is hydrogen, C₁-C₈-alkyl optionally substituted by cyano, amino, nitro, carboxy, C₁-C₈-alkoxy, a C₃-C₁₅-carbocyclic group, C₆-C₁₅-aromatic carbocyclic group, or by a 4- to 12-membered heterocyclic group having at least one ring heteroatom selected from nitrogen, oxygen and sulphur,
or R¹⁰ is a C₃-C₁₅-carbocyclic group, C₆-C₁₅-aromatic carbocyclic group, or a 4- to 12-membered heterocyclic group having at least one ring heteroatom selected from nitrogen, oxygen and sulphur;
R¹¹ is hydrogen, C₁-C₈-alkyl, C₁-C₈-alkyl-C₁-C₈-alkoxy or C₁-C₈-alkyl-O-R¹⁵;
R¹² is a C₃-C₁₅-carbocyclic group or C₆-C₁₆-aromatic carbocyclic group;
R¹³ is C₁C₈-alkyl or a C₃-C₁₅-carbocyclic group or C₆-C₁₅-aromatic carbocyclic group;
R¹⁴ is hydrogen, a C₃-C₁₅-carbocyclic group, C₆-C₁₅-aromatic carbocyclic group, C₁-C₈-alkenyl, or C₁-C₈-alkyl optionally substituted by a C₃-C₁₅-carbocylic group or C₆-C₁₅-aromatic carbocyclic group;
R¹⁵ is a C₃-C₁₅-carbocyclic group or C₆-C₁₅-aromatic carbocyclic group; and
where each C₃-C₁₅-carbocyclic group is optionally substituted by halo (e.g. fluoro, chloro or bromo), cyano, hydroxy, amino, nitro, carboxy, C₁-C₈-alkyl (e.g. methyl or ethyl), halo-C₁-C₈-alkyl, C₁-C₈-alkoxy, C₁-C₈-alkylcarbonyl, C₁-C₈-alkylsulfonyl, -SO₂NH₂, a C₃-C₁₅-carbocyclic group and a 4- to 12-membered heterocyclic group having at least one ring heteroatom selected from nitrogen, oxygen and sulphur and each C₆-C₁₅-aromatic carbocyclic group is optionally substituted by halo (e.g. fluoro, chloro or bromo), cyano, hydroxy, amino, nitro, carboxy, C₁-C₈-alkyl (e.g. methyl or ethyl), halo-C₁-C₈-alkyl, C₁-C₈-alkoxy, C₁C₈-alkylcarbonyl, C₁-C₈-alkylsulfonyl, -SO₂NH₂, a C₃-C₁₅-carbocyclic group and a 4-to 12-membered heterocyclic group having at least one ring heteroatom selected from nitrogen, oxygen and sulphur;
(B) a beta-2 adrenoceptor agonist selected from salmeterol and formoterol, or pharmaceutically acceptable salts thereof; and
(C) a corticosteroid selected from mometasone furoate and a compound of formula II 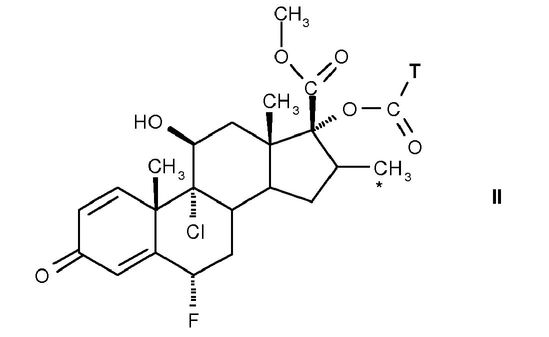 where T is a monovalent cyclic organic group having from 3 to 15 atoms in the ring system;
for simultaneous, sequential or separate administration in the treatment of an inflammatory or obstructive airways disease.

5. A medicament according to claim 1 or 2 where said corticosteroid is fluticasone propionate.

6. A medicament according to claim 1 or 2 where said corticosteroid is budesonide.

7. A medicament according to claim 3 or 4 where said corticosteroid is mometasone furoate.

8. A medicament according to claim 3 or 4 where said corticosteroid is a compound of formula II.

9. A medicament according to claim 8, wherein said compound of formula II comprises T is a heterocyclic aromatic group having a 6 membered heterocyclic ring with one or two ring nitrogen atoms, the heterocyclic ring being unsubstituted or substituted by one or two substituents selected from halogen, cyano, hydroxyl, C₁-C₄-acyloxy, amino, C₁-C₄ alkylamino, di-(C₁-C₄-alkyl)amino, C₁-C₄-alkyl, hydroxy-C₁-C₄-alkyl, halo-C₁-C₄-alkyl C₁-C₄-alkoxy, or C₁-C₄-alkylthio and the heterocyclic ring being optionally fused to a benzene ring.

10. A medicament according to claim 9 wherein said compound of formula II comprises T is 5-methyl-2-thienyl, N-methyl-2-pyrrolyl, cyclopropyl, 2 -furyl, 3-methyl-2-furyl, 3-methyl-2 -thienyl, 5-methyl-3-isoxazolyl, 3,5 -dimethyl-2-thienyl, 2,5 -dimethyl-3 -furyl, 4-methyl-2-furyl, 4-(dimethylamino)phenyl, 4-methylphenyl, 4-ethyl-phenyl, 2-pyridyl, 4-pyrimidyl or 5-methyl 2-pyrazinyl or the indicated 16-methyl group has the beta conformation and R is cyclopropyl.

11. A medicament according to claim 10, wherein said compound of formula II is 3-methyl-thiophene-2-arboxylic acid (6S,9R,10S,11S,13S,16R,17R)-9-chloro-6-fluoro-11-hydroxy-17-methoxycarbonyl-10,13,16-trimethyl-3-oxo-6,7,8,9,10,11,12,13,14,15,16,17-dodecahydro-3H-cyclopenta-[a]phenanthren-17 -yl ester.

12. A medicament according to either claim 1, 2, 3 or 4, which is a pharmaceutical composition comprising a mixture of effective amounts of (A), (B) and (C) optionally together with at least one pharmaceutically acceptable carrier.

13. A medicament according to claim 2 or 4, in which (A) is a compound of formula I in free or salt or solvate form
where, R¹ and R³ are each independently a C₃-C₁₅-carbocyclic group, C₆-C₁₅-aromatic carbocyclic group, or a 4- to 12-membered heterocyclic group having at least one ring heteroatom selected from nitrogen, oxygen and sulphur;
R₂ is hydroxyl;
R⁴ is C₁C₈-alkyl substituted by -CO-NR⁹R¹⁰;
R⁹ is hydrogen or C₁C₄-alkyl; and
R¹⁰ is a 4- to 12-membered heterocyclic group having at least one ring heteroatom selected from nitrogen, oxygen and sulphur;
where each C₃-C₁₅-carbocyclic group is optionally substituted by halo (e.g. fluoro, chloro or bromo), cyano, hydroxy, amino, nitro, carboxy, C₁-C₈-alkyl (e.g. methyl or ethyl), halo-C₁-C₈-alkyl, C₁-C₈-alkoxy, C₁-C₈-alkylcarbonyl, C₁-C₈-alkylsulfonyl, -SO₂NH₂, a C₃-C₁₅-carbocyclic group and a 4- to 12-membered heterocyclic group having at least one ring heteroatom selected from nitrogen, oxygen and sulphur and each C₆-C₁₅-aromatic carbocyclic group is optionally substituted by halo (e.g. fluoro, chloro or bromo), cyano, hydroxy, amino, nitro, carboxy, Ci-C₈-alkyl (e.g. methyl or ethyl), halo-C₁-C₈-alkyl, C₁-C₈-alkoxy, C₁-C₈-alkylcarbonyl, C₁-C₈-alkylsulfonyl, -SO₂NH₂,a a C₃-C₁₅-carbocyclic group and a 4-to 12-membered heterocyclic group having at least one ring heteroatom selected from nitrogen, oxygen and sulphur.

14. A medicament according to claim 2 or 4, in which (A) is (*R*)-3-(2-Hydroxy-2,2-diphenyl-acetoxy)-1-(isoxazol-3-ylcarbamoyl-methyl)-1-azonia-bicyclo-[2.2.2]octane bromide (a quinuclidine) or ((R)3-((R)-2-cylohexyl-2-hydroxy-2-phenylacetoxy)-1-(isoxazol-3-ylcarbamoylmethyl)-1-azoniabicyclo[2.2.2]octane.

15. A medicament according to claims 1 or 3 wherein the glycopyrronium salt is a racemate or a mixture of diastereomers.

16. A medicament according to any one of claims 15 wherein the glycopyrronium salt is a single enantiomer.

17. A medicament according to claim 16 wherein the glycopyrronium salt is glycopyrronium bromide.

18. A medicament according to claim 17 wherein the glycopyrronium salt is (3S,2'R)-3-[(cyclopentyl-hydroxyphenylacetyl)oxy]-1,1-dimethylpyrrolidinium bromide or (3R,2'R)-3-[(cyclopentyl-hydroxyphenylacetyl)oxy]-1,1-dimethylpyrrolidinium bromide.

19. A medicament according to claim 18 wherein the glycopyrronium salt is (35,2'R/3R,2'S)-3-[(cyclopentyl-hydroxyphenylacetyl)oxy]-1,1-dimethylpyrrolidinium bromide

20. A medicament according to any preceding claim, which is in inhalable form and is
(i) an aerosol comprising a mixture of (A), (B) and (C) in solution or dispersion in a propellant;
(ii) a combination of an aerosol containing (A) in solution or dispersion in a propellant, with an aerosol containing (B) in solution or dispersion in a propellant and an aerosol containing (C) in solution or dispersion in a propellant;
(iii) a nebulizable composition comprising a dispersion of (A), (B) and (C) in an aqueous, organic or aqueous/organic medium; or
(iv) a combination of a dispersion of (A) in an aqueous, organic or aqueous/organic medium with a dispersion of (B) in an aqueous, organic or aqueous/organic medium and a dispersion of (C) in an aqueous, organic or aqueous/organic medium.

21. A medicament according to any one of claims 1 to 19, in which (A), (B) and (C) are present in inhalable form as a dry powder comprising fin ely divided (A), (B) and (C) optionally together with at least one particulate pharmaceutically acceptable carrier.

22. A medicament according to claim 20 or 21, in which (A), (B) and (C) have an average particle diameter up to 10 µm.

23. A medicament according to any one of claims 1 to 19, which is
a dry powder in a capsule, the capsule containing a unit dose of (A), a unit dose of (B), a unit dose of (C) and a pharmaceutically acceptable carrier in an amount to bring the total weight of dry powder per capsule to between 5 mg and 50 mg; or
an aerosol comprising (A), (B) and (C) in a propellant, optionally together with a surfactant and/or a bulking agent and/or a co -solvent suitable for administration from a metered dose inhaler adapted to deliver an amount of aerosol containing a unit dose of (A), a unit dose of (B) and a unit dose of (C), or a known fraction of a unit dose of (A), a known fraction of a unit dose of (B) and a known fraction of a unit dose of (C), per actuation.

24. The use of (A), (B) and (C) as defined in any one of claims 1 to 19 in the preparation of a medicament for combination therapy by simultaneous, sequential or separate administration of (A), (B) and (C) in the treatment of an inflammatory or obstructive airways disease.

25. The use of (A), (B) and(C)as defined in any one of claims 1 to 19 in the treatment of asthma or chronic obstructive pulmonary disease.

26. A pharmaceutical kit comprising (A), (B) and (C)as defined in any one of claims 1 to 19 in separate unit dosage forms, said forms being suitable for administration of (A), (B) and (C) in effective amounts, together with one or more inhalation devices for administration of (A), (B) and (C).

27. A medicament comprising, separately or together, (A), (B) and (C) as defined in claims 1, 2, 3 or 4 for simultaneous, sequential or separate administration in the treatment of an inflammatory or obstructive airways disease, substantially as herein described with reference to any one of the Examples.

28. A medicament according to claim 12 further comprising magnesium stearate

29. A medicament according to claim 1, 2, 3, or 4 wherein said beta-2 adrenoreceptor agonist is salmeterol or a pharmaceutically acceptable salt thereof.

30. A medicament according to claim 1, 2, 3, or 4 wherein said beta-2 adrenoreceptor agonist is formoterol or a pharmaceutically acceptable salt thereof.
